# FASCICULE DE BREVET EUROPEEN

(11) **EP 0 839 785 B1**
(45) Date de publication et mention de la délivrance du brevet: **28.03.2001**
(21) Numéro de dépôt: 97402463.0
(22) Date de dépôt: 17.10.1997
(51) Int. Cl.: C07C 17/42, C07C 19/08

(54) **Procédé de stabilisation du pentafluoroéthane**
Verfahren zur Stabilisierung von Pentafluorethan
Process for the stabilisation of pentafluoroethane

(30) Priorité: 04.11.1996 FR 9613400
(43) Date de publication de la demande: 06.05.1998
(73) Titulaire: Atofina, 92800 Puteaux (FR)
(72) Inventeur: Lacroix, Eric, 64480 Ambérieux d'Azergues (FR); Sage, Jean-Marc, 69600 Oullins (FR)

(56) Documents cités:
- EP-A- 0 538 085
- EP-A- 0 539 720
- GB-A- 1 335 618
- US-A- 4 045 503

## Description

La présente invention concerne le domaine des HFC (HydroFluoroCarbone) et a plus particulièrement pour objet un procédé de stabilisation du pentafluoroéthane, connu dans le métier sous la désignation F125 et dont le principal domaine d'application, comme substitut des CFC, est la réfrigération basse température.

L'accès au F125 est possible par différentes voies décrites dans la littérature telles que la fluoration du perchloréthylène ou de l'un de ses dérivés fluorés et l'hydrogénolyse du pentachloroéthane (F115). Le F125 pur est un composé parfaitement stable qui ne subit aucune transformation ou décomposition dans les conditions normales de synthèse, stockage et utilisation (T < 500°C). Cependant, l'obtention d'un F125 de haute pureté n'est pas aisée et, quel que soit le mode de synthèse utilisé, le produit contient comme principales impuretés non seulement du F115, mais aussi diverses oléfines plus ou moins toxiques et qui, notamment le chlorotrifluoroéthylène (F1113), sont difficilement séparables du F125.

A partir de l'étude toxicologique du F125, réalisée au sein du PAFTT III (Program for Alternative Fluorocarbon Toxicity Testing), des spécifications de pureté pour le produit F125 ont été établies. Pour la plupart des oléfines (F1111, F1113, F1114, ...), la spécification pour chacune d'elles est fixée selon sa toxicité à une teneur comprise entre 0 et 1000 ppm. Ainsi, pour les oléfines très toxiques comme le PFIB (PerFluorolsoButène), la concentration tolérée est nettement inférieure à la ppb. A l'inverse, pour des oléfines moins toxiques, la spécification est nettement moins sévère et peut atteindre quelques centaines de ppm.

Ces spécifications en oléfines sont généralement faciles à tenir lorsque l'on utilise les procédés classiques de synthèse du F125 (fluoration, hydrogénolyse). Cependant, certaines de ces oléfines sont difficilement séparables du F125 par simple distillation et c'est pourquoi, dans les produits commerciaux, on note souvent leur présence et notamment celle du F1113. Actuellement, suivant les fournisseurs, le F125 commercialisé présente une teneur en F1113 qui oscille entre 5 et 90 ppm, le plus souvent entre 20 et 80 ppm.

Jusqu'à présent, la stabilisation d'HFC n'a pas semblé nécessaire et on ne s'est intéressé qu'à celle des HCFC (HydroChloroFluoroCarbone) comme le 1,1-dichloro-1-fluoroéthane (F141b) et le 1,1-dichloro-2,2,2-trifluoroéthane (F123). Ainsi, a été décrite dans le brevet FR 2 682 377 la stabilisation du F141b contenant des traces de 1,1-dichloroéthylène (F1130a) au moyen d'un hydrocarbure éthylénique contenant au moins 4 atomes de carbone; l'emploi de l'α-méthylstyrène ou du nitrométhane pour stabiliser le F141b est revendiqué dans le brevet EP 539 719. mousses en présence de polyols, le brevet EP 508 449 préconise l'addition de stabilisants nitrés comme le nitrométhane. La demande WO 92/17559 revendique la stabilisation du F123 au moyen d'un phénol, d'un composé aromatique ou d'un époxyde.

Il a maintenant été trouvé que certaines des oléfines présentes à faibles teneurs dans un F125 commercial pouvaient se décomposer en présence d'oxygène. Cette décomposition peut avoir lieu même dans des conditions très douces, comparables aux conditions usuelles de stockage ou d'utilisation du F125 (température ambiante, système fermé), et se traduit par la présence d'acidité conduisant à un F125 hors spécification.

Pour éviter la formation d'acidité et limiter ainsi les risques de dégradation du matériel fonctionnant avec des mélanges frigorigènes à base de F125, l'élimination totale des oléfines dans le F125 est a priori une solution séduisante ; cependant, l'élimination totale du F1113 dans le F125 par distillation est difficile, voire impossible. L'objectif "zéro oléfine" dans le F125 ne peut donc être atteint qu'au prix d'une étape supplémentaire de purification coûteuse puisqu'il est nécessaire d'avoir recours à des traitements chimiques ou à des épurations physiques.

Selon la présente invention, ce problème est résolu en ajoutant à un F125 contenant des oléfines, (de préférence des oléfines perhalogénées), une quantité suffisante d'au moins un piège à radicaux et/ou un piège à acidité. Il s'est en effet avéré que cet ajout permet de stabiliser un F125 de façon durable et peu coûteuse.

L'ajout d'un piège à radicaux qui bloque les réactions radicalaires en chaîne permet d'éviter la décomposition des oléfines. D'autre part, l'ajout d'un piège à acidité évite que la réaction de décomposition s'autoaccélère avec la formation d'acidité. Ainsi, le F125 reste à pH neutre et la décomposition des oléfines présentes, notamment du F1113, est minime, voire nulle.

Comme pièges à radicaux, on peut utiliser à titre non limitatif les molécules présentant un système de doubles liaisons conjuguées telles que, par exemple, les composés aromatiques, les diènes et les dérivés pyrroliques. Comme exemples non limitatifs de tels composés, on peut mentionner le styrène, l'a-méthylstyrène, le phénol, le 4-méthoxyphénol (EMHQ), le butadiène, l'isoprène, le 3-méthyl-1,2-butadiène, le 1,3-pentadiène, les terpènes et le N-méthylpyrrole.

Le piège à acidité peut, à titre non limitatif, être choisi parmi les amines et les époxydes. Comme exemples d'amines on peut mentionner la triéthylamine et la tributylamine, et comme exemples d'époxydes l'oxyde de butylène et le 1,2-époxyhexane.

Parmi les stabilisants précités, on préfère utiliser plus particulièrement l'α-méthylstyrène, l'oxyde de butylène, la triéthylamine, la tributylamine, l'isoprène ou l'EMHQ.

La quantité de stabilisant à ajouter au F125 dépend bien évidemment de la teneur en impuretés oléfiniques du F125 à traiter. La proportion de stabilisant peut donc aller de quelques dizaines de ppm à quelques pour cent. Généralement, on ajoute une quantité de stabilisant qui, exprimée en équivalents molaires, correspond à un rapport : stabilisant/oléfines présentes dans le F125 compris entre 0,05 et 5, de préférence entre 0,1 et 2. Ainsi, pour un F125 commercial dont la teneur totale en oléfines est généralement très inférieure à 500 ppm en poids, l'ajout de 0,1 % en poids de stabilisant s'avère largement suffisant.

Le stabilisant peut être introduit dans le F125 soit directement dans les bacs de stockage de l'unité de production, soit lors du conditionnement. Pour éviter tout risque de décomposition, les bacs de stockage sont inertés et il est préférable d'introduire le (ou les) stabilisant(s) dès le remplissage de ces bacs.

Les exemples suivants illustrent l'invention sans la limiter.

### EXEMPLES 1 à 8

Dans un tube scellé (volume: 8 ml), on introduit 1,5 g de F125, 500 ppm de F1113, la quantité de stabilisant indiquée dans le tableau (sauf les exemples 6 et 7 réalisés sans stabilisant) et de l'air à raison de 5 % molaire par rapport au F125 (sauf l'exemple 7 réalisé sans air et sans stabilisant).

Chaque tube est stocké à 80°C pendant 48 heures. Après ce laps de temps, le tube scellé est refroidi à la température de l'azote liquide et mis en communication avec une bouteille en acier (volume: 20 ml) préalablement mise sous vide et maintenue à la température de l'azote liquide. On casse ensuite le haut du tube et on réchauffe doucement le tube jusqu'à la température ambiante pour récupérer les gaz par piégeage dans l'éprouvette métallique.

On récupère ainsi dans l'éprouvette 1,5 g du gaz qu'on analyse ensuite par chromatographie gazeuse. Les résultats sont consignés dans le tableau suivant.

Les exemples 1 à 5 montrent que la présence d'un stabilisant inhibe la décomposition du F1113 et l'apparition d'acidité, et ceci malgré des conditions assez sévères (80°C). L'exemple 6 montre que l'ajout d'un stabilisant est nécessaire et l'exemple 7 que l'air est responsable de la décomposition des oléfines et de l'apparition d'acidité.

| **EXEMPLE** | **AIR %** | **STABILISANT** | | **TENEUR EN F1113 APRES L'ESSAI** |
|---|---|---|---|---|
| | | **Nom** | **Quantité** | |
| 1 | 5 | Oxyde de butylène | 0,7 mg 466 ppm | 500 ppm |
| 2 | 5 | α-méthylstyrène | 0,6 mg 400 ppm | 500 ppm |
| 3 | 5 | tributylamine | 0,8 mg 533 ppm | 500 ppm |
| 4 | 5 | triéthylamine | 0,4 mg 266 ppm | 500 ppm |
| 5 | 5 | EMHQ | 0,3 mg 200 ppm | 500 ppm |
| 6 comparatif | 5 | néant | - | 40 ppm |
| 7 comparatif | 0 | néant | - | 500 ppm |
| 8 comparatif | 5 | Nitrométhane | 0,3 mg 200 ppm | 400 ppm |

### EXEMPLES 9 ET 10

Un F125 commercial contenant seulement 14 ppm de F1113 a été stocké pendant 30 jours à température ambiante et en présence d'air (5 % en volume par rapport au F125).

Au F125 de l'exemple 9 on a ajouté 25 ppm de triéthylamine alors que l'exemple 10 a été effectué avec un F125 ne contenant pas de stabilisant.

Après 30 jours de stockage, le F125 stabilisé (exemple 9) n'a montré aucune évolution du produit alors que, pour celui de l'exemple 10, on a noté une diminution de la teneur en F1113 (passage de 14 ppm à 1 ppm) et l'apparition d'acidité (16,5 mg/l exprimé en HCI).

## Revendications

1. Procédé de stabilisation d'un pentafluoroéthane (F125) contenant des oléfines, caractérisé en ce que l'on ajoute audit F125, comme stabilisant, au moins un piège à radicaux et/ou un piège à acidité dans une quantité, exprimée en équivalents molaires, correspondant à un rapport stabilisant/oléfines compris entre 0,05 et 5, de préférence entre 0,1 et 2.

2. Procédé selon la revendication 1 pour la stabilisation d'un F125 contenant des oléfines perhalogénées.

3. Procédé selon la revendication 1 pour la stabilisation d'un F125 contenant du chlorotrifluoroéthylène (F1113).

4. Procédé selon l'une des revendications 1 à 3, dans lequel le piège à radicaux est choisi parmi les molécules présentant un système de doubles liaisons conjuguées.

5. Procédé selon la revendication 4 dans lequel on utilise l'α-méthylstyrène, le 4-méthoxyphénol ou l'isoprène.

6. Procédé selon l'une des revendications 1 à 3, dans lequel le piège à acidité est choisi parmi les amines et les époxydes, de préférence parmi la triéthylamine, la tributylamine et l'oxyde de butylène.

## Claims

1. Process for stabilization of a pentafluoroethane (F125) containing olefins, characterized in that at least one radical scavenger and/or one acidity scavenger is/are added, as stabilizer, to the said F125 in a quantity, expressed in molar equivalents, corresponding to a stabilizer/olefins ratio of between 0.05 and 5, preferably of between 0.1 and 2.

2. Process according to Claim 1 for the stabilization of an F125 containing perhalogenated olefins.

3. Process according to Claim 1 for the stabilization of an F125 containing chlorotrifluoroethylene (F1113).

4. Process according to one of Claims 1 to 3, in which the radical scavenger is chosen from molecules containing a system of conjugated double bonds.

5. Process according to Claim 4 in which α-methylstyrene, 4-methoxyphenol or isoprene is employed.

6. Process according to one of Claims 1 to 3, in which the acidity scavenger is chosen from amines and epoxides, preferably from triethylamine, tributylamine and butylene oxide.

## Patentansprüche

1. Verfahren zur Stabilisierung eines Olefine enthaltenden Pentafluorethans (F125), dadurch gekennzeichnet, daß man dem genannten F125 als Stabilisator mindestens einen Radikalfänger und/oder einen Säuregradsenker in einer Menge zugibt, die definiert in Moläquivalenten einem Verhältnis Stabilisator/Olefinen zwischen 0,05 und 5, vorzugsweise zwischen 0,1 und 2, entspricht.

2. Verfahren nach Anspruch 1 zur Stabilisierung eines perhalogenierte Olefine enthaltenden F125.

3. Verfahren nach Anspruch 1 zur Stabilisierung eines Chlortrifluorethylen (F1113) enthaltenden F125.

4. Verfahren nach einem der Ansprüche 1 bis 3, in dem der Radikalfänger unter Molekülen gewählt ist, die ein System aus konjugierten Doppelbindungen haben.

5. Verfahren nach Anspruch 4, in dem man α-Methylstyrol, 4-Methoxyphenol oder Isopren verwendet.

6. Verfahren nach einem der Ansprüche 1 bis 3, in dem der Säuregradsenker unter Aminen und Epoxiden, vorzugsweise unter Triethylamin, Tributylamin und Butenoxid, ausgewählt ist.
